# EUROPÄISCHE PATENTSCHRIFT

(11) **EP 1 575 906 B1**
(45) Veröffentlichungstag und Bekanntmachung des Hinweises auf die Patenterteilung: **17.11.2010**
(21) Anmeldenummer: 03789264.3
(22) Anmeldetag: 13.12.2003
(51) Int. Cl.: C07C 263/10, B01D 53/70, C01B 7/07

(54) **AUFTRENNUNG EINES STOFFGEMISCHES AUS CHLORWASSERSTOFF UND PHOSGEN**
SEPARATION OF A SUBSTANCE MIXTURE CONSISTING OF HYDROGEN CHLORIDE AND PHOSGENE
DIVISION D'UN MELANGE DE GAZ CHLORHYDRIQUE ET DE PHOSGENE

(30) Priorität: 19.12.2002 DE 10260084
(43) Veröffentlichungstag der Anmeldung: 21.09.2005
(73) Patentinhaber: BASF SE, 67056 Ludwigshafen (DE)
(72) Erfinder: SOHN, Martin, 68229 Mannheim (DE); STROEFER, Eckhard, 68163 Mannheim (DE); NEVEJANS, Filip, B-9170 St.Gillis-Waas (BE); PENZEL, Ulrich, 01945 Tettau (DE); PALLASCH, Hans-Jürgen, 67169 Kallstadt (DE); VAN DEN ABEEL, Peter, B-2930 Brasschaat (BE); DEBERDT, Filip, B-2812 Muizen (BE); JACOBS, Jan D., Baton Rouge, LA 70810 (US); MACKENROTH, Wolfgang, 67089 Bad Dürkheim (DE)
(86) Internationale Anmeldenummer: PCT/EP2003/014188
(87) Internationale Veröffentlichungsnummer: WO 2004/056758

(56) Entgegenhaltungen:
- US-A- 2 764 607
- US-A- 3 142 535
- US-A- 3 544 611
- US-A- 3 574 695
- US-B1- 6 479 690

## Beschreibung

Die Erfindung betrifft ein Verfahren zur partiellen oder vollständigen Auftrennung eines gasförmigen oder ganz oder teilweise kondensierten Gemisches aus Chlorwasserstoff (HCl), Phosgen (COCl₂) sowie gegebenenfalls Inertgasen und/oder Leichtsiedern, wie Stickstoff, Nebenprodukte aus der Phosgensynthese wie Kohlenmonoxid, Chlor, Methan, Tetrachlorkohlenstoff (CCl₄) und Chloroform (CHCl₃) sowie anderen leichtsiedenden Komponenten) sowie absichtlich oder unabsichtlich enthaltenem Lösungsmittel wie z.B. chlorierte, vorzugsweise aromatische Kohlenwasserstoffe wie Dichlorbenzol, Chlorbenzol, Trichlorbenzol, aromatische oder aliphatische Kohlenwasserstoffe wie Toluol, Xylol, Benzol, Pentan, Hexan, Heptan, Octan, Cyclohexan, Biphenyl, Ketonen wie 2-Butanon, Methylisobutylketon, Ester wie Diethylisophtalate, Ethylacetat, Butylacetat, Nitrile wie Acetonitril, oder Sulfolan, wie es typischerweise bei der Umsetzung von aliphatischen oder aromatischen Aminen mit Phosgen zu den entsprechenden Isocyanaten anfällt.

Ähnliche Gemische aus Phosgen und Chlorwasserstoff fallen beispielsweise auch bei der Herstellung von chlorierten organischen Verbindungen, wie Säurechloriden oder Chlorformiaten, an.

Aromatische Isocyanate, wie TDI (Toluylendiisocyanat) und MDI (Methylendiphenyldiisocyanat) beziehungsweise PMDI (Polymethylenpolypheylenpolyisocyanat), sowie aliphatische Isoyanate, wie HDI (Hexamthylendiphenyldiisocyanat) und IPDI (Isophorondiisocyanat) werden durch Umsetzung der entsprechenden Amine mit Phosgen großtechnisch und global verteilt hergestellt. Bei diesen Synthesen wird Chlorwasserstoff als Nebenprodukt, im Prozeß meist gasförmig, frei. Außerdem wird bei der Reaktion in der Regel ein mehr oder weniger großer Phosgenüberschuß eingesetzt, so daß das eingesetzte Phosgen trotz hoher chemischer Ausbeuten bei der Isocyanatproduktion nicht vollständig mit dem Amin abreagiert. Das überschüssige Phosgen fällt in der Regel zumindest zum Teil zusammen mit dem freigesetzten Chlorwasserstoff meist gasförmig an und wird mit diesem teilweise oder gänzlich von der Reaktionsmischung abgetrennt. Je nach Abtrennung können dabei auch noch mehr oder weniger große Mengen Lösungsmittel mit der Mischung aus Chlorwasserstoff und Phosgen abgetrennt oder mitgerissen werden. Auch können eventuell aus der Phosgensynthese mitgeschleppte oder absichtlich oder durch den Prozeß bedingt zugesetzte Inertgase und Leichtsieder wie Stickstoff in der Mischung aus Chlorwasserstoff und Phosgen enthalten sein. Gegebenenfalls. können diese aber auch vor oder nach der Mischung aus Chlorwasserstoff und Phosgen abgetrennt werden. Es ist zum wirtschaftlichen Betrieb einer Isocyanatsynthese unabdingbar, die Mischung aus Chlorwasserstoff und Phosgen aufzutrennen, das überschüssige Phosgen in die Isocyanatsynthese oder zu einer anderen Verwendung zurückzuführen sowie den abgetrennten Chlorwasserstoff einer weiteren Verwendung zuzuführen oder zu entsorgen. Diese weitere Verwendung des Chlorwasserstoffs können z.B. eine Oxichlorierung zur Herstellung von Vinylchlorid, ein Deacon-Prozeß zur Wiedergewinnung von Chlor, welches wieder in der Phosgensynthese eingesetzt werden kann, oder die Herstellung von wäßriger Salzsäure sein. Der in der Isocyanatherstellung anfallende Chlorwasserstoff muß insbesondere im Falle der Verwendung für die Oxichlorierung oder für den Deacon-Prozess dezidierte Reinheitsanforderungen erfüllen, so daß für die Trennung von Phosgen und Chlorwasserstoff Verfahrensvarianten gewählt werden müssen, bei denen sichergestellt wird, daß der Chlorwasserstoff nach der Trennung und Aufarbeitung nur noch geringe Mengen von Nebenprodukten, insbesondere chlorierte Lösungsmittel wie Dichlorbenzol oder Chlorbenzol enthält. Diese chlorierten aromatischen Verbindungen desaktivieren in der Regel den Katalysator in den Oxichlorierungen. Des weiteren wirken sie, wie Phosgen, störend bei der Produktion von wäßriger Salzsäure für die Nahrungsmittelindustrie oder andere Anwendungen. Von Restphosgenmengen im Chlorwasserstoff geht außerdem ein nicht unerhebliches Gefahrenpotential und Gesundheitsrisiko aus.

Die kontinuierliche Herstellung von organischen Isocyanaten durch Reaktion von primären organischen Aminen mit Phosgen ist vielfach beschrieben und wird im großtechnischen Maßstab durchgeführt. Sie ist beispielsweise beschrieben im Kunststoffhandbuch, Band 7 (Polyurethane), 3. neubearbeitete Auflage, Carl Hanser Verlag, München-Wien, S. 76ff (1993). In der üblichen Ausführungsform des Verfahrens erfolgt die Auftrennung des entstandenen Gemisches aus Phosgen und Chlorwasserstoff meist durch partielle oder vollständige Kondensation des Phosgens, durch Destillation oder durch Absorption von Phosgen in einem inerten Lösungsmittel.

In der Regel wird Phosgen aus dem Gemisch aus Phosgen und Chlorwasserstoff zunächst partiell auskondensiert. Daher wird die Auftrennung meist entweder unter hohem Druck, vorzugsweise bei 10-50 bar, oder bei sehr niedrigen Temperaturen, vorzugsweise um -30°C, durchgeführt. Bei hohen Drücken kann Phosgen leicht und kostengünstig, beispielsweise unter Verwendung von Kühlwasser, auskondensiert werden. Dies ist wirtschaftlich vorteilhaft, allerdings birgt der hohe Druck ein großes Sicherheitsrisiko im Falle einer Leckage, wie schon in DE 3212510 beschrieben. Niedrige Temperaturen zur Trennung von Phosgen und Chlorwasserstoff gehen in der Regel mit niedrigen Drücken einher, allerdings ist zur Erzeugung der niedrigen Temperaturen ein erheblicher Energieaufwand erforderlich. Des weiteren ist gemäß dem Henry'schen Gesetz die Löslichkeit von Chlorwasserstoff in Phosgen sehr hoch, so daß das Rückführphosgen größere Mengen Chlorwasserstoff enthält. Dieser kann bei der Phosgenierung zur Bildung von Aminhydrochlorid in der Phosgenierung des Amins führen. Die Phosgenierung von Aminhydrochloriden erfordert längere Verweilzeiten und höhere Phosgenüberschüsse und impliziert mithin einen höheren Phosgen-Holdup.

Eine Möglichkeit zur Trennung der genannten Gemische ist die Destillation. In US 3544611 wird ein Verfahren zur Herstellung von organischen Isocyanaten bei einem Druck im Bereich von 10 bis 50 bar beschrieben. Überraschenderweise wurde gefunden, daß die Durchführung der Reaktion bei höheren Drücken, mindestens 10 atü, zu höheren Ausbeuten an Isocyanat führt. Des weiteren wird durch höhere Drücke die Trennung von Phosgen und Chlorwasserstoff erleichtert. Der erste Reaktionsschritt zur Isocyanatherstellung, die Reaktion zwischen Amin und Phosgen zum Zwischenprodukt Carbamoylchlorid, wird in einem Mischkreis durchgeführt. Der zweite Reaktionsschritt, die Zersetzung des Carbamoylchlorids zum Isocyanat, erfolgt in einer dem Mischkreis nachgeschalteten Reaktionskolonne, wobei am Kopf der Kolonne eine Mischung aus Phosgen und Chlorwasserstoff anfällt. Daraus wird Phosgen zweistufig auskondensiert, wobei der erste Kühler zur partiellen Kondensation mit Kühlwasser und der zweite zur möglichst vollständigen Kondensation mit Sole bei möglichst tiefer Temperatur betrieben wird. Das auskondensierte Phosgen wird auf den Kopf der Kolonne zurückgeführt. An einem Flüssigabzug im Verstärkungsteil der Kolonne wird dann das Phosgen wieder entnommen und der Reaktion im Mischkreis wieder zugeführt.

Die Restphosgenabtrennung aus der Reaktionsmischung, welche am Sumpf der Reaktionskolonne entnommen wird, erfolgt in einer weiteren Kolonne. In letzterer wird Phosgen über Kopf genommen, analog zur ersten Kolonne zweistufig mit Kühlwassser und Sole kondensiert und in den Mischkreis zur Reaktion zurückgeführt. Da die Reaktion in der Reaktionskolonne abgeschlossen wird, enthält dieses am Kopf der zweiten Kolonne entnommene Phosgen nur noch den Chlorwasserstoff, der in der Reaktionsmischung beim Eintritt in die Restphosgenabtrennkolonne gelöst war.

Nachteilig an diesem Verfahren ist, daß nur ein Teil des Phosgens zur Minimierung des Phosgenverlustes aus dem Chlorwasserstoff auskondensiert werden kann. Eine vollständige Abtrennung durch Kondensation ohne Rektifikation ist nicht möglich, so daß größere Mengen Phosgen verloren gehen und des weiteren der erzeugte Chlorwasserstoff stark mit Phosgen verunreinigt ist. Sowohl das am Seitenabzug der Reaktionskolonne abgezogene als auch das in der Restphosgenabtrennkolonne über Kopf genommenen Phosgen enthalten Chlorwasserstoff. Die Konzentration ergibt sich jeweils gemäß dem Henry'schen Gesetz aus Druck, Temperatur und Zusammensetzung der Gas- und Flüssigphase. Nachteilig sind außerdem die hohen Drücke, von denen im Falle einer Leckage ein hohes Gefährdungspotential ausgeht.

In US 3544611 wird noch eine alternative Trennung von Phosgen und Chlorwasserstoff bei Drücken von 15 bar bis 50 bar oder höher unter Kondensation und Abzug von flüssigem Chlorwasserstoff beschrieben. Die Brüden aus dem Isocyanatsynthesereaktor werden in eine kleine Seitenkolonne geleitet, an deren oberem Ende flüssiger Chlorwasserstoff abgezogen wird. Analog zur oben beschriebenen Verfahrensvariante wird die Reaktion zum Isocyanat in einem Mischkreis und einer nachfolgenden Reaktionskolonne durchgeführt. Ebenfalls analog wird in einem Dephlegmator am Kopf der Reaktionskolonne Phosgen größtenteils kondensiert und auf die Kolonne zurückgeführt. Die am Dephlegmator abgezogenen Mischung aus Phosgen und Chlorwasserstoff wird der oben beschriebenen Seitenkolonne zugeführt, die beim gleichen Druck betrieben wird wie die Reaktionskolonne. Am Kopfkondensator dieser Seitenkolonne wird Chlorwasserstoff kondensiert und zum einen als Rücklauf auf diese Kolonne zurückgeführt und zum anderen flüssig entnommen zur weiteren Verwendung. Die Kolonne besitzt einen reinen Verstärkungsteil, aber keinen Abtriebsteil und Sumpfumlaufverdampfer und dient somit nur zur Reinigung des Chlorwasserstoffes. Die Reinigung des am Sumpf dieser Kolonne abgezogenen Phosgens erfolgt durch Rückführung auf den Kopf der Reaktionskolonne. An einem Flüssigabzug der Reaktionskolonne wird, analog zur oben beschriebenen ersten Verfahrensvariante, flüssiges Phosgen abgezogen und der Amin-Phosgen-Reaktion im Mischkreis zugeführt. Ebenfalls analog zur ersten beschriebenen Verfahrensvariante wird das Restphosgen in einer der Reaktionskolonne nachfolgenden Restphosgenabtrennkolonne über Kopf genommen, zweistufig kondensiert und in den Mischkreis zur Umsetzung in der Amin-Phosgenreaktion zurückgeführt.

Nachteilig an diesem Verfahren ist, daß die zweite Kolonne zur Abtrennung und Reinigung des Chlorwasserstoffes in der Seitenkolonne auf dem gleichem Druckniveau wie die Reaktionskolonne, in der die Umsetzung zum Isocyanat stattfindet, betrieben wird. Dadurch erfolgt eine unerwünschte Kopplung von Reaktions- und Aufarbeitungsteil. Vorteilhafter kann die Trennung von Phosgen und Chlorwasserstoff bei höheren oder niedrigeren Drücken als die Reaktion durchgeführt werden. So bedeuten in US 3544611 hohe Drücke, die vorteilhaft für die Trennung von Phosgen und Chlorwasserstoff sind, auch hohe Temperaturen im Reaktionsteil der Isocyanatsynthese, die zu Nebenreaktionen führen und die Ausbeute an Isocyanat erheblich schmälern. Des weiteren wird die Reaktionskolonne zur Reinigung des Phosgens durch Entfernung von Resten des Chlorwasserstoffs im Verstärkungsteil der Reaktionskolonne verwendet. Dies ist ebenfalls eine unerwünschte Kopplung. Des weiteren kommt es zur Sättigung des zur Amin-Phosgen-Reaktion vorgesehenen, am Flüssigabzug der Reaktionskolonne entnommenen Phosgens mit Chlorwasserstoff. Es stellt sich gemäß des Gas-Flüssig-Gleichgewichts die dem Druck und der Temperatur auf diesem Boden entsprechende Konzentration des Chlorwasserstoffs in der Flüssigphase, dem Phosgen, ein. Da der Druck und die Temperatur in der Reaktionskolonne durch die Reaktion vorgegeben sind, können nicht unabhängig von der Reaktion günstigere Bedingungen eingestellt werden, bei denen beispielsweise die Konzentration des Chlorwasserstoffs im Phosgen deutlich niedriger ist.

Nachteilig an der zweiten Verfahrensvariante ist vor allem die Kondensation des Chlorwasserstoffs, die trotz der hohen Drücke bei sehr tiefen Temperaturen unter hohem Energieverbrauch erfolgt. Von den hohen Drücken geht im Falle einer Leckage ein hohes Gefährdungspotential aus. Energetisch nachteilig ist auch, daß Chlorwasserstoff als Flüssigkeit gewonnen wird. In der Regel wird der in der Isocyanatproduktion gewonnene Chlorwasserstoff gasförmig weiterverarbeitet, beispielsweise in einer Oxichlorierung zur Ethylendichloridherstellung, so daß der Chlorwasserstoff unter Energieaufwand wieder verdampft werden muß.

In GB 827376 wird ein kontinuierliches Verfahren zur Herstellung aromatischer Isocyanate durch Reaktion eines Amins in freier Form oder als Salz, welches sich leicht zum freien Amin zersetzen läßt, und einer Lösung von Phosgen bei einem Druck von größer als 3*10⁵ Pa beschrieben, dadurch gekennzeichnet, daß die Reaktanten unter Vermischung gleichzeitig in das untere Ende eines vertikalen Rohrreaktors eingeführt werden, in dem die Reaktionsprodukte schnell zum oberen Ende aufsteigen. Die Flüssigphase wird in einem Behälter aufgefangen, aus dem sie zur Isolierung des Isocyanats abgenommen wird. Dieser Behälter kann ein Phasentrennapparat sein, der unter dem gleichen Druck betrieben wird, über ein Überlaufrohr mit dem Flüssigauslaß kommuniziert und ein Drosselventil im Flüssigauslaß besitzt. Die im Behälter abgetrennte Flüssigkeit wird einer Kolonne zugeführt, die unter Atmosphären- oder Überdruck und erhöhter Temperatur betrieben wird, wodurch restliches Phosgen und Chlorwasserstoff gasförmig über Kopf abgetrennt werden. Aus der im Behälter abgetrennten Mischung aus Phosgen und Chlorwasserstoff wird das überschüssige Phosgen auskondensiert, und der so abgetrennte Chlorwasserstoff entspannt und abgeführt. Die Reaktanten werden mit einer gemeinsamen oder zwei getrennten Pumpen dem Rohreaktor zugeführt oder in einer Venturi-Mischdüse vermischt und von dort in den Rohrreaktor eingebracht. Die beschriebene Temperatur im Rohrreaktor beträgt 80-200°C, der Druck ist größer 3*10⁵ Pa, maximal dem Dampfdruck der Reaktionsmischung und vorzugsweise 15 bis 20*10⁵ Pa.

Nachteilig ist die sehr geringe Reinheit des Chlorwasserstoffs, da durch einstufige Kondensation bei den bevorzugten Drücken von 15 bis 20*10⁵ Pa erhebliche Mengen Phosgen im Chlorwasserstoff verbleiben, die bei einer weiteren Verwendung des Chlorwasserstoffs zur Oxichlorierung, einem Deacon-Prozeß oder zur Herstellung wäßriger Salzsäure sehr störend sind. Nachteilig ist auf der hohe Chlorwasserstoff-Gehalt des auskondensierten Phosgens, das ohne weitere Aufreinigung in die Reaktion rezykliert wird.

In US 3381025 wird ein Verfahren zur Herstellung von Methylen-bis(4-phenylisocyanat) (MDI), 2,4-Toluylendiisocyanat (TDI), Polymethylen-poly(phenylisocyanat) (polymeres MDI) beschrieben. Dabei macht man sich das Prinzip der Siedekühlung zunutze, das heißt es wird ein Gemisch aus Lösungsmittel, Phosgen und Chlorwasserstoff bei Reaktionstemperatur verdampft. Vorteilhaft ist hierbei, daß der Partialdruck des Phosgens durch die Anwesenheit des Lösungsmittels reduziert wird, und sich daher Phosgen leichter kondensieren läßt. Die Kondensation des Phosgens zusammen mit dem als Lösungsmittel verwendeten Chlorbenzol erfolgt durch Abkühlung der Gasmischung. Die Phosgen-Lösungsmittelmischung wird in die Reaktion zurückgeführt, der Chlorwasserstoff mit Restmengen an Phosgen wird in einem Absorber vernichtet. Es wird eine 22 gew.-%-ige Mischung von Phosgen in Chlorbenzol gewonnen. Nachteilig an diesem Verfahren ist die Abtrennung des Phosgen-Lösungsmittelgemisches durch Abkühlung auf die sehr tiefe Temperatur von -30°C. Des weiteren ist der hohe Verlust von Phosgen mit dem ausgetragenen Chlorwasserstoff von 3,4 Mol.-% Phosgen im Chlorwasserstoff wirtschaftlich nachteilig und sicherheitstechnisch kritisch. Für die meisten Anwendungen wie eine Oxichlorierung, einen Deacon-Prozeß oder zur Salzsäureherstellung kann dieser Chlorwasserstoffstrom nicht verwendet werden. Ein weiterer Nachteil ist der hohe Chlorwasserstoffgehalt des erhaltenen Phosgen-Lösungsmittelgemisches, da die Löslichkeit von Chlorwasserstoff in diesem Gemisch bei tiefen Temperaturen besonders hoch ist.

In US 3381025 wird die Phosgenierung eines organischen primären Amins zum entsprechenden Isocyanat zweistufig bei einer Temperatur <60°C in der ersten Stufe und von 100 bis 190°C in der zweiten Stufe durchgeführt. Aus der zweiten Reaktionsstufe wird eine Mischung aus dem inerten Lösungsmittel, überschüssigem Phosgen und dem gebildeten Chlorwasserstoff abgezogen und der Chlorwasserstoff durch Abkühlen der Mischung auf -20°C von dieser abgetrennt. Die erhaltene kalte Mischung aus Phosgen und Lösungsmittel wird in die erste Reaktionsstufe zurückgeführt. Nachteilig ist hier die erforderliche starke Abkühlung des Gemisches, die hohe Energiekosten und Investitionen für Kälteaggregate erforderlich macht. Die Abkühlung ist insbesondere dann nachteilig, wenn die Mischung wieder auf Reaktionstemperatur aufgeheizt werden muß. Nachteilig ist des weiteren der hohe Chlorwasserstoffgehalt des erhaltenen Phosgen-Lösungsmittelgemisches.

In WO 99/11597 wird die Trennung von Chlorwasserstoff und Phosgen unter Druck in einer Kolonne, die einem Reaktor zur Chlorformiatherstellung nachgeschaltet ist, beschrieben. Der Reaktor wird bei Drücken von 2 bis 60 bar, bevorzugt von 6 bis 40 bar, betrieben. Hohe Drücke werden vorteilhaft zur Trennung von Phosgen und Chlorwasserstoff erkannt, da die Kondenstoren dann nicht bei tiefen Temperaturen betrieben werden müssen.

Eine weitere Möglichkeit der Trennung von Phosgen und Chlorwasserstoff ist die Wäsche. In SU 1811161 wird ein Verfahren zur Herstellung von Isocyanaten durch die Reaktion von Aminen mit Phosgen beschrieben. Für die Umsetzung mit dem Amin wird eine Lösung von Phosgen im Lösungsmittel Chlorbenzol durch Absorption von gasförmigem Phosgen in Chlorbenzol hergestellt. Der Vorteil liegt in der Einsparung von Energiekosten, da nur eine einmalige Kondensation und keine weitere Verdampfung des Phosgens erforderlich ist. Amin und Phosgenlösung werden in einem Reaktor zur Reaktion gebracht. In einem Phasenscheider werden Gas- und Flüssigphase, enthaltend Isocyanat, Chlorbenzol und Phosgen, getrennt. Die dem Phasenscheider entnommenen Gasphase aus Chlorwasserstoff, Phosgen und Chlorbenzol wird partiell kondensiert und einem Absorber zugeführt. Die Flüssigphase wird einer Strippkolonne zugeführt, in der Chlorwasserstoff und Phosgen über Kopf abgetrennt, partiell kondensiert und dann ebenfalls dem Absorber zugeführt werden. Der nicht kondensierbare Anteil aus der Strippkolonne wird in die Phosgensynthese zugeführt. Im Absorber wird das Phosgen mit Chlorbenzol, welches vom Isocyanat/ Chlorbenzolgemisch abdestilliert wurde, absorbiert. Der Phosgen/ Chorbenzolstrom mit etwa 70 Gew.-% Phosgen wird mit einem Phosgen/Chlorbenzolstrom aus der Phosgensynthese mit nachfolgender Absorption in Chlorbenzol vereinigt und als Phosgenlösung zur Amin-Phosgen-Reaktion verwendet. Der über im Absorber Kopf gehende Chlorwasserstoff-Strom wird einer weiteren Behandlung zugeführt, da er noch etwa 4 Gew.-% Phosgen enthält.

Über Temperaturen und Drücke, bei denen der Absorber oder der Reaktor betrieben werden, wird in dem Dokument keine Aussage gemacht. Nachteilig an dem in SU 1811161 beschriebenen Verfahren ist die geringe Reinheit sowohl des erhaltenen gasförmigen Chlorwasserstoffs mit etwa 4 Gew.-% Phosgen als auch die flüssige Phosgen/Chlorbenzollösung, die größere Mengen Chlorwasserstoff aufgrund der tiefen Temperaturen des zum Waschen verwendeten Chlorbenzols enthält. Aufgrund der hohen Phosgenkonzentration kann der Chlorwasserstoffstrom nicht zu den üblichen Zwecken, wie Oxichlorierung, Deacon oder Salzsäureherstellung eingesetzt werden. Es ist allgemein bekannt und u.a. auch publiziert in I.I. Konstantinov, A.I. Kormucheshkina, Zhurnal Prikladnoi Khimii, 49 (3), S. 596-599, 1976, sowie DE 3212510, US 3574695 oder DE 3323882), daß Chlorwasserstoff mit Aminen in der Phosgenierung zu Hydrochloriden reagiert, deren Umsetzung zum Isocyanat erheblich längere Verweilzeiten, Phosgenüberschüsse und Temperaturen erfordert. Außerdem wird eine erhöhte Nebenproduktbildung beobachtet. Die Sättigung der Phosgenlösung für die Reaktion mit Chlorwasserstoff führt somit zu größeren Apparaten, höherem Phosgenholdup, höherem Energieverbrauch und verringerter Ausbeute.

In EP 0570799 wird ein Verfahren beschrieben, in dem die Reaktion zwischen Amin und Phosgen zum Isocyanat in der Gasphase durchgeführt wird. Die Trennung des entstehenden Chlorwasserstoff/Phosgengemisches wird mittels Kondensation, Absorption von Phosgen in einem bei einer Temperatur von -10°C bis 8°C gehaltenen inerten Lösungsmittel, beispielsweise Chlorbenzol oder Dichlorbenzol, oder durch Adsorption und Hydrolyse an Aktivkohle beschrieben.

In US 3226410 wird ein kontinuierliches Verfahren zur Herstellung aromatischer Isocyanate durch Einmischen eines Stromes eines aromatischen Amins in einen Phosgenstrom in einem Rohrreaktor bei Reynoldszahlen von größer 2100, bevorzugt 5000-2000000, und Temperaturen von 60 bis 90°C, bevorzugt 80 bis 85°C, beschrieben. Die Phosgenmenge beträgt mindestens 1 mol, bevorzugt 6 bis 12 mol, pro mol Amin. Die Reaktionslösung wird dann, gegebenenfalls nach Vorerwärmung, in einen zweiten Reaktor, der als Kessel oder Kolonneausgeführt ist, überführt, der eine Temperatur von 110 bis 135°C, bevorzugt 110 bis 120°C besitzt. Die Aminkonzentration beträgt 2 bis 25 Gew.-%, bevorzugt 5 bis 10 Gew.-%, die Phosgenkonzentration 10 bis 100 Gew.-%, bevorzugt 10 bis 60 Gew.-%. Der Druck, mit dem der Phosgenstrom in den Rohrreaktor überführt wird, beträgt 50 bis 170 psig. Aus dem zweiten Reaktor wird die Flüssigphase, enthaltend Isocyanat, Lösungsmittel, relativ geringe Mengen Nebenprodukte, Chlorwasserstoff und Phosgen gelöst im Lösungsmittel getrennt von der Gasphase, enthaltend Chlorwasserstoff, Lösungsmittel, Phosgen und Spuren des Isocyanats, abgezogen. Als Lösungsmittel werden chlorierte Kohlenwasserstoffe, die inert sind und einen geringeren Siedepunkt als das Isocyanat besitzen, verwendet. Besonders bevorzugt ist Chlorbenzol. An den zweiten Reaktor mit einem Druck von 45 psig oder höher schließt sich ein Verweilzeitbehälter und ein Puffergefäß an, aus dem standgeregelt in eine Kolonne zum Entfernen von überschüssigem Phosgen gefahren wird. Phosgen, Chlorwasserstoff und Lösungsmittel werden über Kopf entnommen und in den Phosgenbehälter zurückgeführt. Das Sumpfprodukt, bestehend aus Isocyanat und Lösungsmittel wird in die einstufige destillative Lösungsmittelabtrennung gefahren. Das vom Isocyanat abgetrennte Lösungsmittel wird zur Absorption des restlichen Phosgens aus dem Chlorwasserstoffstrom verwendet. Das im zweiten Reaktor und im Pufferbehälter abgezogene Phosgen wird zweistufig kondensiert und in den Phosgenbehälter zurückgeführt. Das nichtkondensierte Phosgen/Chlorwasserstoffgemisch wird in einen Wäscher gefahren, der mit in der Lösungsmittelabtrennung gewonnenem Lösungsmittel beaufschlagt wird. Das nicht absorbierte Gas, hauptsächlich Chlorwasserstoff, wird anschließend in einem Absorber mit Wasser zu wäßriger Salzsäure umgesetzt.

Weiterhin bekannt ist die chemische Trennung von Phosgen und Chlorwasserstoff. In WO 9831662 wird die Herstellung von 3-(isocyanatomethyl)-1,6-hexamethylendiisocyanat durch Phosgenierung des entsprechenden Amins beschrieben. Als Chlorwasserstoff-Fänger wird ein tertiäres Amin zugesetzt, welches als Aminhydrochlorid ausfällt. Die Maische wird in Anwesenheit eines Chlorwasserstoff-Fängers destilliert. Auch in DE 1233854 wird die gebildete Chlorwasserstoff durch den Zusatz ein tertiäres Amins gebunden. Der Zusatz organischer Stickstoffbasen wird auch in DE 3000524 und US 3211776 angewendet.

In JP 09208589 wird zum Abfangen des in der Reaktion gebildeten Chlorwasserstoffs eine Alkalimetall- oder Erdalkalimetallverbindung verwendet, bevorzugt ein schwach saures Salz oder Oxid wie Kalziumoxid.

In WO 9530663 wird die Synthese von funktionalisierten 1,3,5-Triazinisocyanaten beschrieben, in der ebenfalls Chlorwasserstoff in der Reaktion zumindest teilweise chemisch gebunden wird.

Nachteilig ist bei diesen Verfahren der Feststoffanfall, der verfahrenstechnisch schwierig zu handhaben ist. Die Aufarbeitung ist erheblich erschwert, da die gebildeten Ammoniumsalze häufig auskristallisieren. Des weiteren macht die Einführung eines zusätzlichen Hilfsstoffes das Verfahren unwirtschaftlich. Durch die Hilfsstoffe können außerdem diverse Nebenprodukte in der Phosgenierung entstehen, die die Qualität des gewünschten Isocyanats mindern.

Die Aufgabe der vorliegenden Erfindung ist es daher, Mischungen aus Phosgen und Chlorwasserstoff kostengünstig, das heißt mit niedrigem Energieverbrauch, und unter Sicherheitsaspekten betrachtet mit geringem Gefahrenpotential in möglichst reinen Chlorwasserstoff und reines Phosgen aufzutrennen.

Verbleibt bei der Trennung von Phosgen und Chlorwasserstoff gelöster Chlorwasserstoff im Rückführphosgen, so reagiert dieser in der ersten Stufe der Phosgenierung direkt mit dem freien Amin zu Aminhydrochlorid. Die Reaktionsgeschwindigkeit der Hydrochloridphosgenierung ist aber erheblich kleiner als die des freien Amins, wie in I.I. Konstantinov, A.I. Kormucheshkina, Zhurnal Prikladnoi Khimii, 49 (3), S. 596-599, 1976) beschrieben. Außerdem bedarf die Umsetzung von Aminhydrochloriden mit Phosgen zum Isocyanat höherer Temperaturen und neigt daher, wie in GB 1212249 beschrieben, zu verstärkter Nebenproduktbildung, insbesondere zur Bildung von Harnstoffen. Die Vermeidung der Bildung von Aminhydrochloriden führt somit zu kleineren Apparaten, geringerem Phosgenholdup, niedrigerem Energieverbrauch und höherer Ausbeute. Da die Löslichkeit von Aminhydrochloriden in den entsprechenden Reaktionsgemischen und auch in den meisten kommerziell erhältlichen Lösungsmitteln sehr gering ist, tritt durch Hydrochloridbildung zudem das Problem des Feststoffanfalls auf. Daher ist es wichtig, daß die zur Reaktion gebrachten Lösungen von Amin und Phosgen keinen gelösten Chlorwasserstoff enthalten.

Der durch die Reaktion von Amin mit Phosgen in situ gebildete Chlorwasserstoff kann in einer Folgereaktion mit dem noch vorhandenem Amin ebenfalls Aminhydrochlorid bilden. Es wurde allerdings gefunden, daß ein deutlicher Unterschied in der Größenordnung der Hydrochloridbildung und auch der Abreaktion mit Phosgen darin besteht, ob der Chlorwasserstoff direkt zu Reaktionsbeginn mit dem in hoher Konzentration vorliegenden Amin in Kontakt gebracht wird und praktisch quantitativ Aminhydrochlorid bildet, welches als Feststoff ausfällt, oder ob erst im Reaktionsverlauf gebildeter Chlorwasserstoff mit dem dann nur noch in sehr geringer Konzentration vorliegenden Amin zu Aminhydrochlorid reagiert. Die Reaktion zwischen Amin und Phosgen ist auch bei tiefen Temperaturen extrem schnell, so daß die Aminkonzentration zu Reaktionsbeginn stark sinkt. Aminhydrochlorid aus in situ gebildetem Chlorwasserstoff zeigt eine hohe Übersättigung in der Reaktionsmischung, wobei das bereits in höherer Konzentration vorliegende Isocyanat als Lösungsvermittler wirkt. Es reagiert daher im Gegensatz zu aus Amin und Chlorwasserstoff direkt gebildetem und als Feststoff ausgefallenem Aminhydrochlorid relativ schnell mit Phosgen zum Carbamoylchlorid.

Gegenstand der vorliegenden Erfindung ist somit ein Verfahren zur partiellen oder vollständigen Auftrennung eines Stoffgemisches bestehend aus Chlorwasserstoff und Phosgen, gegebenenfalls Lösungsmittel, Leichtsiedern und Inerten, wie sie üblicherweise bei der Herstellung von Isocyanaten durch Umsetzung von Aminen mit Phosgen anfallen, dadurch gekennzeichnet, daß zunächst eine partielle oder vollständige Kondensation von Phosgen, dann eine Destillation oder Strippung in einer Kolonne zur Entfernung des Chlorwasserstoffes aus dem Sumpfprodukt Phosgen und anschließend eine Wäsche des Kopfproduktes Chlorwasserstoff mit dem Prozeßlösungsmittel zur Absorption des Phosgens im Prozeßlösungsmittel durchgeführt wird. Zur Entfernung von Lösungsmittelresten aus dem Phosgen und/oder Chlorwasserstoff kann anschließend deren Nachreinigung mittels Adsorption, beispielsweise auf Aktivkohle, oder durch andere geeignete Methoden erfolgen.

Im erfindungsgemäßen Verfahren erfolgt die teilweise oder vollständige Auftrennung eines Stoffgemisches aus Chlorwasserstoff und Phosgen und gegebenenfalls den genanten anderen Komponenten durch partielle Kondensation, gegebenenfalls mehrstufig und. bei verschiedenen Temperatur- und Druckniveaus, anschließende Destillation oder Strippung in einer Kolonne zur Entfernung von Chlorwasserstoff aus dem Phosgen und schließlich Absorption des im Chlorwasserstoffstrom verbliebenen Phosgens im Prozesslösungsmittel. Dieses ist Chlorbenzol, Dichlorbenzol, Mischungen der beiden oder Toluol.

Am Kopf des Absorbers fällt weitgehend reiner Chlorwasserstoff an, der einer weiteren Verwendung zugeführt werden kann. Der am Sumpf der Destillationskolonne anfallende Phosgenstrom wird in die Isocyanatsynthese zurückgeführt, vorzugsweise zur Umsetzung mit dem Amin in der ersten Stufe, beispielsweise in einem statischen Mischer. Er kann aber auch in einen anderen Apparat des Reaktions- oder Aufarbeitungsteils geführt werden. Der am Sumpf des Absorbers anfallende Phosgen/Waschmittelstrom wird ebenfalls in die Isocyanatsynthese zurückgeführt, das heißt in den ersten oder einen der folgenden Reaktoren oder in eine Kolonne zur Phosgenabtrennung oder zur Aufarbeitung des Reaktionsgemisches. Insbesondere kann dieser Strom als Rücklauf auf eine Kolonne, beispielsweise eine Reaktionskolonne, verwendet werden. Dies ist besonders deswegen vorteilhaft, da er neben Phosgen und dem Waschmittel noch Chlorwasserstoff enthält, das bei Rückführung in den ersten Reaktor zur Bildung von Aminhydrochlorid führen würde. Gegebenenfalls kann der den Absorber verlassende Chlorwasserstoffstrom einer Nachreinigung, insbesondere einer Adsorption auf Aktivkohle, einer Druckwechseladsorption, einer weiteren Wäsche, oder anderen Nachreinigungsverfahren, unterworfen werden. Dadurch können Reste von störenden Komponenten, insbesondere von chlorierten Kohlenwasserstoffen, wie den Lösungsmitteln Dichlorbenzol oder Chlorbenzol, aus dem Chlorwasserstoffstrom entfernt werden.

Die für das erfindungsgemäße Verfahren eingesetzte Mischung aus Phosgen und Chlorwasserstoff enthält, wie oben ausgeführt. üblicherweise neben Chlorwasserstoff und Phosgen gegebenenfalls Lösungsmittel wie Dichlorbenzol, Chlorbenzol, Toluol oder andere sowie gegebenenfalls Leichtsieder und Inerte wie Stickstoff, Kohlenmonoxid, Methan, Tetrachlorkohlenstoff oder Chloroform.

Die partielle Kondensation von Phosgen aus dem anfallenden Gemisch, enthaltend Chlorwasserstoff, Phosgen, gegebenenfalls Lösungsmittel und Inerte, erfolgt ein- oder vorzugsweise mehrstufig bei Temperaturen von -40°C, erreichbar mittels Kältemittel, bis 40°C, erreichbar mittels Kühlwasser, je nach dem Druck im Reaktionsteil. Die Destillation zur Entfernung von Chlorwasserstoff aus dem Rückführphosgen wird bei einer Sumpftemperatur von 5 bis 150°C, bevorzugt 5 bis 50°C, einem Kopfdruck von 1 bis 35 bar, bevorzugt 1,5 bis 4,0 bar und einer Kopftemperatur von -20°C bis 30°C, bevorzugt von -10°C bis 0°C, durchgeführt. Alternativ kann der Chlorwasserstoff auch durch Strippen mit einem Inertgas wie Stickstoff, dem Prozeßlösungsmitteldampf, Phosgen oder einem anderen gasförmigen oder zu verdampfenden Stoff aus dem Rückführphosgen entfernt werden. Die Kopftemperatur des Absorbers beträgt -40°C bis 10°C, bevorzugt von -15°C bis 0°C, die Sumpftemperatur -10°C bis 30°C, bevorzugt 0 bis 10°C, und der Kopfdruck 1 bis 35 bar, bevorzugt 1,5 bis 4,0 bar Der Absorptionsmittelstrom kann vorzugsweise vorher mit Chlorwasserstoff gesättigt werden, damit die Sättigung des Absorptionsmittels mit Chlorwasserstoff nicht im Wäscher stattfindet. Die freiwerdende Lösungswärme von Chlorwasserstoff im Absorptionsmittelstrom kann dann extern in einem Wärmetauscher abgeführt werden. Zur Abführung der freiwerdenden Lösungswärme von Chlorwasserstoff im Absorptionsmittelstrom kann alternativ auch eine Zwischenkühlung am Absorber verwendet werden.

Die vorgeschaltete partielle Kondensation ist aus energetischen Gründen vorteilhaft, da die Kondensation stufenweise bei verschiedenen Temperatur- und gegebenenfalls Druckniveaus erfolgen kann. Die nachfolgende Destillation liefert am Sumpf einen weitgehend chlorwasserstofffreien Phosgenstrom, so daß sich bei der Umsetzung mit Amin erheblich weniger Aminhydrochlorid bilden kann.

Der Vorteil der dann folgenden Absorption von Phosgen aus dem Chlorwasserstoffstrom ist die Einsparung von Energiekosten, da keine Kondensation und keine weitere Verdampfung des im Chlorwasserstoffstrom verbliebenen Phosgens erforderlich ist. Besondere energetische Einsparungen können erzielt werden, wenn der phosgenhaltige Austrag des Absorbers dem Phosgenierreaktor als Feed oder ggf. einer Reaktionskolonne oder einer Kolonne zur Phosgenabtrennung oder zur Aufarbeitung des Reaktionsgemisches als Rücklauf zugeführt wird, wodurch ggf. in den letzteren Fällen der Kolonnenkopfkondensator eingespart und auf die partielle oder vollständige Kondensation des Brüdenstromes zur Erzeugung eines Rücklaufes verzichtet werden kann. Dadurch wird der Energieverbrauch erheblich gesenkt.

Die Erfindung soll an dem nachfolgenden Beispiel näher erläutert werden.

### Beispiel:

Die Trennung eines Chlorwasserstoff / Phosgengemisches erfolgte durch partielle Kondensation, Destillation in einer Glockenbodenkolonne und anschließend Absorption in einem nachgeschalteten Wäscher, wodurch am Sumpfaustrag der Destillationskolonne sehr reines Phosgens und am Kopfaustrag des Wäschers reiner Chlorwasserstoff auf ökonomisch günstige Weise und gleichzeitig bei niedrigen Drücken und damit vermindertem Gefahrenpotential hergestellt werden kann. Aus dem so abgetrennten Chlorwasserstoff werden Lösungsmittelreste mittels Adsorption entfernt.

### 1) Partielle Kondensation von Phosgen:

Die partielle Kondensation von Phosgen aus einem Strom aus einer Isocyanatsynthese wurde in zwei aufeinanderfolgenden Wärmetauschern bei 33°C (Kühlwasser) und bei -15°C (Sole) durchgeführt. Die eingesetzte Chlorwasserstoff /Phosgenmischung betrug 3,26 kg/h. Der eingesetzte Strom enthielt Phosgen, Chlorwasserstoff, Chlorbenzol, die für eine Isocyanatsynthese typischen Leichtsieder und Inerte. Die Zusammensetzung war 2,43 kg/h Phosgen (74,6 Gew.-%), 0,235 kg/h Chlorwasserstoff (7,2 Gew.-%), 0,56 kg/h Chlorbenzol (17,3 Gew.-%), 0,0106 kg/h CCl₄ (0,3 Gew.-%), 0,0169 kg/h CHCl₃ (0,5 Gew.-%), 0,001 kg/h Kohlendioxid (0,02 Gew.-%) und geringen Mengen Stickstoff (4 ppm). Das erhaltende Gemisch wurde zweiphasig in den Mittelteil der nachfolgenden Destillationskolonne geführt.

### 2) Destillation zur Entfernung von Chlorwasserstoff aus dem Phosgen zur Rückgewinnung möglichst reinen Phosgens:

Es wurde eine Glockenbodenkolonne mit 33 Glockenböden verwendet, wobei der Abtriebsteil 22 Glocken und der Verstärkungsteil 11 Glocken besaß. Der Durchmesser der Kolonne betrug 55 mm. Der Kopfdruck betrug 2,5 bar abs. und die Sumpftemperatur 38°C. Die Kopftemperatur der Kolonne betrug -9°C. Als Verdampfertyp wurde ein Robertverdampfer eingesetzt, als Kopfkondensator ein Rohrbündelapparat mit 13 Rohren.

Das aus der partiellen Kondensation erhaltene Gemisch wurde zwischen Abtriebs- und Verstärkungsteil der Kolonne eingespeist. Die Feedtemperatur war 24,5°C. Der Zulauf, die Chlorwasserstoff/Phosgen/Chlorbenzolmischung, wurde zwischen Abtriebs- und Verstärkungsteil zugegeben.

Es wurde ein Gesamtstrom von 5,53 kg/h am Sumpf abgezogen, der 4,9 kg/h Phosgen (89 Gew.-%), 0,0005 kg/h Chlorwasserstoff (100 ppm), 0,57 kg/h Chlorbenzol (10 Gew.-%), 0,02 kg/h CHCl₃ (0,3 Gew.-%) und 0,01 kg/h CCl₄ (0,2 Gew.-%) enthielt. Das am Sumpf der Kolonne abgezogene Phosgen/Chlorbenzolgemisch enthielt damit nur in geringen Spuren Chlorwasserstoff , das heißt, das Phosgen war bezüglich Chlorwasserstoff sehr rein. Das inerte Lösungsmittel Chlorbenzol störte bei einer Rückführung in den Reaktionsteil nicht.

Der am Kopf der Kolonne abgezogene Phosgen/Chlorwasserstoffstrom wurde am Kopfkondensator partiell kondensiert und als Rücklauf auf die Kolonne zurückgeführt. Die Rücklaufmenge betrug 0,2 kg/h, wobei die Rücklauftemperatur -20°C war. Im nichtkondensierbaren Strom des Kopfkondensators von 1,24 kg/h lag folgende Zusammensetzung vor: 0,92 kg/h Chlorwasserstoff (74 Gew.-%), 0,32 kg/h Phosgen (26 Gew.-%), 0,2 Gew.-% Kohlendioxid, 0,001 kg/h Kohlenmonoxid, (800 ppm) und geringe Mengen Stickstoff (140 ppm).

### 3) Absorber (Wäscher):

Entfernung des Phosgens durch Wäsche des Chlorwasserstoffstroms.

Es wurde eine Packungskolonne mit einem Durchmesser von 30 mm verwendet, die mit drei Schüssen mit 3x3 mm Maschendrahtringen gefüllt war. Die Schütthöhe betrug je 630 mm pro Schuss. Der Kopfdruck betrug 2,2 bar abs. und die Sumpftemperatur 6°C. Die Kopftemperatur der Kolonne betrug -8°C. Die Kolonne war mit einem Kopfkondensator ausgestattet. Im Kopf der Kolonne wurde ein Demister zur Vermeidung von Tröpfchenmitriß installiert.

Der zugeführte Strom der Chlorwasserstoff /Phosgenmischung betrug 1,24 kg/h und besaß die o.g. Zusammensetzung. Die Feedtemperatur war -20°C. Auf den Kopf des Wäschers wurde ein -25°C kalter Chlorbenzolstrom von 1,32 kg/h gegeben. Die Zusammensetzung betrug 1,3 kg/h Chlorbenzol (99,6 Gew.-%), 0,003 kg/h CCl₄ (0,2 Gew.-%) und 0,002 kg/h CHCl₃ (0,2 Gew.-%). Zur Abführung der freiwerdenden Lösungswärme von Chlorwasserstoff im Wasch-Chlorbenzol wurde eine Zwischenkühlung verwendet. Der Zwischenkühler wurde bei -30°C betrieben.

Der am Kopf des Wäschers anfallende Brüdenstrom wurde dem Kopfkondensator zugeführt, an dem vor allem mitgerissenen Tröpfchen abgeschieden wurden. Der kondensierbare Anteil wurde in den Sumpf des Wäschers zurückgeführt. Der nichtkondensierbare Anteil von 0,87 kg/h hatte eine Zusammensetzung von 0,86 kg/h Chlorwasserstoff (99,5 Gew.-%), 0,001 kg/h Phosgen (0,1 Gew.-%), 0,001 kg/h Chlorbenzol (0,1 Gew.-%), 0,001 kg/h Kohlendioxid(0,1 Gew.-%), 0,001 kg/h Kohlenmonoxid (0,1 Gew.-%) und 0,0005 kg/h N₂ (500 ppm) und jeweils geringe Mengen CCl₄ und CHCl₃ Phosgen (je 80 ppm). Der Kopfkondensator wurde bei -30°C betrieben. Der so gewonnene Chlorwasserstoffstrom ist sehr rein (99,9 Gew.-%). Die am Sumpf der Kolonne abgezogene Chlorbenzol/Phosgenmischung von 1,69 kg/h hat die Zusammensetzung von 0,32 kg/h Phosgen (19 Gew.-%), 1,3 kg/h Chlorbenzol (78 Gew.-%), 0,05 kg/h Chlorwasserstoff (3 Gew.-%), 0,0027 kg/h CCl₄ (0,2 Gew.-%), 0,002 kg/h CHCl₃ (0,1 Gew.-%) und geringe Mengen Stickstoff (60 ppm).

### 4) Nachreinigung von Chlorwasserstoff aus der Chlorwasserstoff/ Phosgentrennung

Der gasförmige Austrag am Kopf des der Absorbers wurde auf einen Aktivkohlefilter geleitet, in dem Reste von Phosgen und Chlorbenzol auf der Aktivkohle adosrbiert wurden. Es wurde ein sehr reiner Chlorwasserstoffstrom erhalten, in dem mit Inline IR- oder GC-Analyse kein Phosgen oder Chlorbenzol mehr nachweisbar war.

## Patentansprüche

1. Verfahren zur partiellen oder vollständigen Auftrennung eines Stoffgemisches bestehend aus Chlorwasserstoff und Phosgen, ggf. Lösungsmittel und ggf. Leichtsieder und Inerte, wie sie üblicherweise bei der Herstellung von Isocyanaten durch Umsetzung von Aminen mit Phosgen anfallen, **dadurch gekennzeichnet, daß** zunächst eine partielle oder vollständige Kondensation von Phosgen, dann eine Destillation oder Strippung in einer Kolonne zur Entfernung des Chlorwasserstoffes aus dem Sumpfprodukt Phosgen und anschließend eine Wäsche des Kopfproduktes Chlorwasserstoff mit Chlorbenzol, Dichlorbenzol, Mischungen der beiden oder Toluol zur Absorption des Phosgens in Chlorbenzol, Dichlorbenzol, Mischungen der beiden oder Toluol durchgeführt wird.

2. Verfahren nach Anspruch 1, **dadurch gekennzeichnet, dass** die partielle oder vollständige Kondensation von Phosgen bei Temperaturen von -40°C bis 40°C und bei Drücken von 1 bis 35 bar, bevorzugt 3 bis 16 bar, durchgeführt wird.

3. Verfahren nach Anspruch 1 oder 2, **dadurch gekennzeichnet, dass** die Destillation zur Entfernung von Chlorwasserstoff aus Phosgen bei einer Sumpftemperatur von 5 bis 150°C, bevorzugt 5 bis 50°C, einem Kopfdruck von 1 bis 35 bar, bevorzugt 1,5 bis 4,0 bar und einer Kopftemperatur von -20°C bis 30°C, bevorzugt von -10°C bis 0°C, durchgeführt wird.

4. Verfahren nach einem der Ansprüche 1 bis 3, **dadurch gekennzeichnet, dass** der Chlorwasserstoff durch Strippen mit einem Inertgas wie Stickstoff, Prozesslösungsmitteldampf, Phosgen oder einem anderen gasförmigen oder zu verdampfenden Stoff aus dem Phosgen entfernt wird.

5. Verfahren nach einem der Ansprüche 1 bis 4, **dadurch gekennzeichnet, dass** die Kopftemperatur des Absorbers -40°C bis 10°C, bevorzugt von -15°C bis 0°C, die Sumpftemperatur -10°C bis 30°C, bevorzugt 0 bis 10°C und der Kopfdruck 1-35 bar, bevorzugt 1,5-4,0 bar beträgt.

6. Verfahren nach einem der Ansprüche 1 bis 5, **dadurch gekennzeichnet, dass** der Absorptionsmittelstrom für die Absorption vorher mit Chlorwasserstoff gesättigt und gegebenenfalls die Kondensationswärme abgeführt wurde.

7. Verfahren nach einem der Ansprüche 1 bis 6, **dadurch gekennzeichnet, dass** die Kondensationswärme von Chlorwasserstoff und Phosgen im Absorptionsmittel durch eine Zwischenkühlung im Absorber abgeführt wird.

8. Verfahren nach einem der Ansprüche 1 bis 7, **dadurch gekennzeichnet, dass** eine Nachreinigung mittels Adsorption, vorzugsweise auf Aktivkohle, durchgeführt wird.

9. Verfahren nach einem der Ansprüche 1 bis 8, **dadurch gekennzeichnet, dass** die Wäsche mit Chlorbenzol erfolgt.

10. Verfahren nach einem der Ansprüche 1 bis 9, **dadurch gekennzeichnet, dass** das am Sumpf der Destillationskolonne gewonnene Phosgen oder die Phosgenlösung in den Reaktionsteil einer Isocyanatsynthese zurückgeführt wird.

11. Verfahren nach einem der Ansprüche 1 bis 10, **dadurch gekennzeichnet, dass** das am Sumpf der Destillationskolonne gewonnene Phosgen oder die Phosgenlösung als Rücklauf auf Destillations- oder Reaktionskolonnen oder als Waschlösung für Absorber oder Wäscher verwendet wird.

12. Verfahren nach einem der Ansprüche 1 bis 11, **dadurch gekennzeichnet, dass** die am Sumpf des Absorbers gewonnene Phosgenlösung in den Reaktionsteil einer Isocyanatsynthese zurückgeführt wird.

13. Verfahren nach einem der Ansprüche 1 bis 12, **dadurch gekennzeichnet, dass** die am Sumpf des Absorbers oder der Waschkolonne gewonnene Phosgenlösung als Rücklauf auf Destillations- oder Reaktionskolonnen oder als Waschlösung für den Absorber oder Wäscher verwendet wird.

14. Verfahren nach einem der Ansprüche 1 bis 13, **dadurch gekennzeichnet, dass** der gewonnene Chlorwasserstoff nachfolgend komprimiert wird.

15. Verfahren nach einem der Ansprüche 1 bis 14, **dadurch gekennzeichnet, dass** der gewonnene Chlorwasserstoff nachfolgend für eine Ethylendichlorid- (Vinylchlorid-) herstellung oder für einen Deaconprozeß verwendet wird.

## Claims

1. A process for the partial or complete separation of a mixture comprising hydrogen chloride and phosgene, possibly solvents and possibly low boilers and inerts as are typically obtained in the preparation of isocyanates by reaction of amines with phosgene, which comprises firstly carrying out a partial or complete condensation of phosgene, then a distillation or stripping step in a column to remove the hydrogen chloride from the bottom product phosgene and subsequently a scrub of the top product hydrogen chloride by means of chlorobenzene, dichlorobenzene, mixtures of the two or toluene solvent to absorb the phosgene in chlorobenzene, dichlorobenzene, mixtures of the two or toluene.

2. The process according to claim 1, wherein the partial or complete condensation of phosgene is carried out at from -40°C to 40°C and pressures of from 1 to 35 bar, preferably from 3 to 16 bar.

3. The process according to claim 1 or 2, wherein the distillation to remove hydrogen chloride from phosgene is carried out at a temperature of the bottom of from 5 to 150°C, preferably from 5 to 50°C, a pressure at the top of from 1 to 35 bar, preferably from 1.5 to 4.0 bar, and a temperature at the top of from -20°C to 30°C, preferably from -10°C to 0°C.

4. The process according to any of claims 1 to 3, wherein the hydrogen chloride is removed from the phosgene by stripping with an inert gas such as nitrogen, process solvent vapor, phosgene or another gaseous or vaporizable substance.

5. The process according to any of claims 1 to 4, wherein the temperature at the top of the absorber is from -40°C to 10°C, preferably from -15°C to 0°C, the temperature at the bottom is from -10°C to 30°C, preferably from 0 to 10°C, and the pressure at the top is 1-35 bar, preferably 1.5-4.0 bar.

6. The process according to any of claims 1 to 5, wherein the absorption medium stream for the absorption has been saturated beforehand with hydrogen chloride and, if desired, the heat of condensation has been removed.

7. The process according to any of claims 1 to 6, wherein the heat of condensation of hydrogen chloride and phosgene in the absorption medium is removed by intermediate cooling in the absorber.

8. The process according to any of claims 1 to 7, wherein an after-purification by means of adsorption, preferably on activated carbon, is carried out.

9. The process according to any of claims 1 to 8, wherein the scrub is carried out using chlorobenzene.

10. The process according to any of claims 1 to 9, wherein the phosgene obtained at the bottom of the distillation column or the phosgene solution is recirculated to the reaction section of an isocyanate synthesis.

11. The process according to any of claims 1 to 10, wherein the phosgene obtained at the bottom of the distillation column or the phosgene solution is used as runback in distillation or reaction columns or as scrubbing solution for absorbers or scrubbers.

12. The process according to any of claims 1 to 11, wherein the phosgene solution obtained at the bottom of the absorber is recirculated to the reaction section of an isocyanate synthesis.

13. The process according to any of claims 1 to 12, wherein the phosgene solution obtained at the bottom of the absorber or the scrubbing column is used as runback for distillation or reaction columns or as scrubbing solution for the absorber or scrubber.

14. The process according to any of claims 1 to 13, wherein the hydrogen chloride obtained is subsequently compressed.

15. The process according to any of claims 1 to 14, wherein the hydrogen chloride obtained is subsequently used for a preparation of ethylene dichloride (or vinyl chloride) or for a Deacon process.

## Revendications

1. Procédé de séparation partielle ou totale d'un mélange de substances constitué de chlorure d'hydrogène et de phosgène, éventuellement d'un solvant et éventuellement de composés de point d'ébullition bas et de composés inertes, tel que formé généralement lors de la fabrication d'isocyanates par réaction d'amines avec du phosgène, **caractérisé en ce qu'**une condensation partielle ou totale du phosgène est tout d'abord réalisée, puis une distillation ou extraction dans une colonne pour éliminer le chlorure d'hydrogène du produit de fond phosgène, et enfin un lavage du produit de tête chlorure d'hydrogène avec du chlorobenzène, du dichlorobenzène, des mélanges des deux ou du toluène pour absorber le phosgène dans du chlorobenzène, du dichlorobenzène, des mélanges des deux ou du toluène.

2. Procédé selon la revendication 1, **caractérisé en ce que** la condensation partielle ou totale du phosgène est réalisée à des températures de -40 °C à 40 °C et à des pressions de 1 à 35 bar, de préférence de 3 à 16 bar.

3. Procédé selon la revendication 1 ou 2, **caractérisé en ce que** la distillation pour éliminer le chlorure d'hydrogène du phosgène est réalisée à une température de fond de 5 à 150 °C, de préférence de 5 à 50 °C, à une pression de tête de 1 à 35 bar, de préférence de 1,5 à 4,0 bar et à une température de tête de -20 °C à 30 °C, de préférence de -10 °C à 0 °C.

4. Procédé selon l'une quelconque des revendications 1 à 3, **caractérisé en ce que** le chlorure d'hydrogène est éliminé du phosgène par extraction avec un gaz inerte tel que l'azote, la vapeur du solvant du procédé, du phosgène ou une autre substance gazeuse ou à vaporiser.

5. Procédé selon l'une quelconque des revendications 1 à 4, **caractérisé en ce que** la température de tête de l'absorbeur est de -40 °C à 10 °C, de préférence de -15 °C à 0 °C, la température de fond est de -10 °C à 30 °C, de préférence de 0 à 10 °C, et la pression de tête est de 1 à 35 bar, de préférence de 1,5 à 4,0 bar.

6. Procédé selon l'une quelconque des revendications 1 à 5, **caractérisé en ce que** le courant d'agent d'absorption pour l'absorption est auparavant saturé avec du chlorure d'hydrogène et la chaleur de condensation est éventuellement dissipée.

7. Procédé selon l'une quelconque des revendications 1 à 6, **caractérisé en ce que** la chaleur de condensation du chlorure d'hydrogène et du phosgène dans l'agent d'absorption est dissipée dans l'absorbeur par un refroidissement intermédiaire.

8. Procédé selon l'une quelconque des revendications 1 à 7, **caractérisé en ce qu'**une purification secondaire par adsorption est réalisée, de préférence sur du charbon actif.

9. Procédé selon l'une quelconque des revendications 1 à 8, **caractérisé en ce que** le lavage a lieu avec du chlorobenzène.

10. Procédé selon l'une quelconque des revendications 1 à 9, **caractérisé en ce que** le phosgène ou la solution de phosgène obtenu au fond de la colonne de distillation est recyclé dans la partie de réaction d'une synthèse d'isocyanate.

11. Procédé selon l'une quelconque des revendications 1 à 10, **caractérisé en ce que** le phosgène ou la solution de phosgène obtenu au fond de la colonne de distillation est utilisé en tant que reflux dans des colonnes de distillation ou de réaction ou en tant que solution de lavage pour l'absorbeur ou l'épurateur.

12. Procédé selon l'une quelconque des revendications 1 à 11, **caractérisé en ce que** la solution de phosgène obtenue au fond de l'absorbeur est recyclée dans la partie de réaction d'une synthèse d'isocyanate.

13. Procédé selon l'une quelconque des revendications 1 à 12, **caractérisé en ce que** la solution de phosgène obtenue au fond de l'absorbeur ou de la colonne de lavage est utilisée en tant que reflux dans des colonnes de distillation ou de réaction ou en tant que solution de lavage pour l'absorbeur ou l'épurateur.

14. Procédé selon l'une quelconque des revendications 1 à 13, **caractérisé en ce que** le chlorure d'hydrogène obtenu est ensuite comprimé.

15. Procédé selon l'une quelconque des revendications 1 à 14, **caractérisé en ce que** le chlorure d'hydrogène obtenu est ensuite utilisé pour une fabrication de dichlorure d'éthylène (chlorure de vinyle) ou pour un procédé Deacon.
